Europäisches Patentamt

European Patent Office

Office européen des brevets

(11) Veröffentlichungsnummer: **0 015 612**
**B1**

(12) **EUROPÄISCHE PATENTSCHRIFT**

(45) Veröffentlichungstag der Patentschrift:
**16.05.84**

(21) Anmeldenummer: **80200167.7**

(22) Anmeldetag: **27.02.80**

(51) Int. Cl.³: **A 61 B 6/00**

(54) Röntgenuntersuchungsgerät mit einer in Tischlängsrichtung verfahrbaren, einen Röntgenstrahler tragenden Säule.

(30) Priorität: **07.03.79 DE 2908914**

(43) Veröffentlichungstag der Anmeldung:
**17.09.80 Patentblatt 80/19**

(45) Bekanntmachung des Hinweises auf die Patenterteilung:
**16.05.84 Patentblatt 84/20**

(84) Benannte Vertragsstaaten:
**DE FR GB IT NL SE**

(56) Entgegenhaltungen:
**FR - A - 1 386 420**
**FR - A - 1 568 600**
**GB - A - 916 178**
**US - A - 2 588 124**
**US - A - 3 244 883**
**US - A - 3 838 286**

(73) Patentinhaber: **Philips Patentverwaltung GmbH,
Billstrasse 80, D-2000 Hamburg 28 (DE)**
(84) Benannte Vertragsstaaten: **DE**

(73) Patentinhaber: **N.V. Philips' Gloeilampenfabrieken,
Groenewoudseweg 1, NL-5621 BA Eindhoven (NL)**
(84) Benannte Vertragsstaaten: **FR GB IT NL SE**

(72) Erfinder: **Christiansen, Dieter, Dorfstrasse 29a,
D-2071 Schönberg (DE)**

(74) Vertreter: **Hartmann, Heinrich, Dipl.-Ing. et al, Philips
Patentverwaltung GmbH
Billstrasse 80 Postfach 10 51 49,
D-2000 Hamburg 28 (DE)**

Röntgenuntersuchungsgerät mit einer in Tischlängsrichtung verfahrbaren, einen
Röntgenstrahler tragenden Säule

Die Erfindung betrifft ein Röntgenuntersuchungsgerät mit einem Grundgestell, einer verschiebbar mit dem Grundgestell verbundenen Patientenlagerungsplatte und einer einen Röntgenstrahler tragenden Säule, die längs einer sich in Tischlängsrichtung erstreckenden Führungsschiene verfahrbar ist.

Ein solches Gerät ist aus der US-A 3 838 286 bekannt und kann u. a. auch zur Anfertigung von Buckyaufnahmen benutzt werden.

Bei modernen Buckygeräten werden Buckyaufnahmen überwiegend dadurch ausgeführt, daß Röntgenstrahler und Laufraster aufeinander ausgerichtet in einer zentralen Position bleiben, während die schwimmende Tischplatte mit dem Patienten darauf verschoben wird. Für diese Aufnahmeverfahren ist es wichtig, daß das Grundgestell in Tischlängsrichtung geringe Abmessungen hat. Dann kann nämlich der Untersucher von der Kopf- oder Fußseite her ganz an das Grundgestell herantreten, wenn die Tischplatte zur Fuß- bzw. Kopfseite hin verschoben ist.

Eine weitere Forderung ist, daß der Röntgenstrahler über einen relativ großen Weg in Tischlängsrichtung und über das Grundgestell hinaus verschiebbar sein muß, z. B. um Aufnahmen mit Hilfe eines an das Kopf- oder Fußende geschobenen Blattfilmwechslers durchführen zu können.

Ein zur Anfertigung von Buckyaufnahmen geeignetes Gerät, das diese Forderungen erfüllt, ist bekannt (z. B. Geräte »ITS« der Firma Machlett Grafax). Die Führungsschiene zur Führung der Säule ist bei diesem Gerät an der Längsseite des Patientenlagerungstisches angeordnet und länger als das Grundgestell des Patientenlagerungstisches. Die kasten- bzw. rahmenförmige Führungseinrichtung ragt also beiderseits des Grundgestells heraus und steht fest im Raum. Das hat den Nachteil, daß die herausstehenden Elemente formgeberisch schlecht in der ansonsten kleinen Einheit unterzubringen sind. Außerdem wird das Herumgehen um das Gerät und das Arbeiten am Patienten an der Fuß- und Stirnseite, und erst recht an der Längsseite, an der die Führungseinrichtung untergebracht ist, erheblich erschwert oder ganz ausgeschlossen, da der Fuß- und Kniebereich über die gesamte Tischlänge blockiert ist.

Nun ist zwar aus der Zeitschrift »Röntgenstrahlen« Heft 41, 1979, Seiten 26 bis 31 auch ein zur Anfertigung von Buckyaufnahmen geeignetes Gerät bekannt, bei dem eine den Zutritt zum Gerät behindernde Führungsschiene nicht erforderlich ist, weil der Röntgenstrahler von einem in Tischlängsrichtung verfahrbaren Deckenstativ getragen wird. Dieses Gerät erfordert jedoch zusätzlich eine an der Decke angebrachte Führungseinrichtung für das Deckenstativ.

Es ist Aufgabe der vorliegenden Erfindung, ein für die Anfertigung von Buckyaufnahmen geeignetes Gerät zu schaffen, das keine zusätzlichen Führungseinrichtungen an der Decke oder an der Wand benötigt, das ebenfalls ein schmales Grundgestell und einen weiten Verschiebebereich der Säule aufweist und darüber hinaus es den Untersucher gestattet, auch von der Seite an das Gerät heranzutreten, an der sich die Führungsschiene befindet.

Diese Aufgabe wird ausgehend von einem Gerät der eingangs genannten Art erfindungsgemäß dadurch gelöst, daß die Führungsschiene in Tischlängsrichtung längs eines Wagens verfahrbar ist, der ebenfalls in Tischlängsrichtung längs eines ortsfesten Rahmens verfahrbar ist, daß zwischen der Führungsschiene und dem Rahmen wenigstens eine am Wagen befestigte Rolle so angeordnet ist, daß bei einer Verschiebung der Führungsschiene die Rolle zwangsläufig auf dem Rahmen abrollt, und daß der Wagen und die Säule so miteinander gekoppelt sind, daß bei einer Verschiebung des Wagens relativ zur Führungsschiene die Säule relativ zur Führungsschiene gegensinnig um dieselbe Strecke verschoben wird.

Bei einem solchen Gerät brauchen die Abmessungen der Führungseinrichtung, die aus dem Wagen, der Führungsschiene und dem Rahmen besteht, nicht größer zu sein als das die Tischplatte tragende Grundgestell des Patientenlagerungstisches, obwohl die Säule auf beiden Seiten über das Grundgestell hinaus in Tischlängsrichtung der Tischplatte verschoben werden kann. Beim Verschieben der Säule bewegen sich die einzelnen Teile der Führungseinrichtung teleskopartig gegeneinander, d. h. der Wagen wird einen Teil der Verschiebungsstrecke gegenüber dem ortsfesten Rahmen verschoben, die Führungsschiene wird gegenüber dem Wagen verschoben und die Säule — bzw. ein mit ihr starr verbundener, sie tragender weiterer Wagen — wird wiederum gegenüber der Führungsschiene verschoben. Alle diese Verschiebungen erfolgen in der gleichen Richtung und addieren sich also, so daß die Gesamtverschiebung der Säule in Tischlängsrichtung größer sein kann als den Abmessungen der Führungsschiene in dieser Richtung entspricht. Dadurch ergibt sich eine sehr kompakte Bauweise, und ein Teil der Längsseite, auf der sich die Führungseinrichtung befindet, ist für den Arzt frei zugänglich.

Es sei an dieser Stelle erwähnt, daß aus der US-A-3 244 883 an sich ein Röntgengerät bekannt ist, bei dem die Röntgenröhre an einer Teleskoparmhalterung befestigt ist und horizontal senkrecht zur Tischlängsrichtung verschoben werden kann.

Dabei verschieben sich die einzelnen Arme der Teleskoparmhalterung in Achsrichtung jeweils um die gleiche Strecke relativ zueinander. Um diese gleichmäßige Verschiebung der einzelnen Teleskoparme zu erreichen, ist ein aufwendiger Mechanismus vorgesehen. Demgegenüber sorgt bei der Erfindung die Rolle am Wagen dafür, daß der Wagen und die Führungsschiene in bezug auf den Rahmen gleichmäßig bewegt werden.

Grundsätzlich könnten die beweglichen Teile auf Rollen oder Schienen am Boden laufen. Dadurch könnte der Untersucher aber unter Umständen mit dem Fuß unter den bewegten Geräteteil geraten. Eine Weiterbildung der Erfindung sieht daher vor, daß die am Wagen befestigte Rolle um eine horizontale Achse drehbar ist und das Gewicht der Führungsschiene trägt, die ihrerseits das Gewicht der auf ihr verfahrbaren Säule trägt. Dabei berührt also nur der ortsfeste Rahmen den Boden. Die beweglichen Teile werden jeweils von dem in Richtung auf das Grundgestell benachbarten bewegten Teile getragen. Die Rolle mit der horizontalen Achse hat dabei zwei Funktionen: Sie trägt die Führungsschiene und damit das Gewicht der von dieser getragenen Säule — bzw. des weiteren, die Säule tragenden Wagens — und sie erzwingt, daß bei einer Verschiebung der Führungsschiene der Wagen die Hälfte der Strecke zurücklegt, die dabei die Führungsschiene zurücklegt, weil die Führungsschiene auf dieser Rolle abrollt, die dadurch ihrerseits auf dem Rahmen abrollt und sich dabei (um die halbe Strecke) in der Verschieberichtung bewegt.

Die Erfindung und ihre Weiterbildungen werden nachstehend anhand der Zeichnung näher erläutert. Es zeigt in schematischer Darstellung

Fig. 1 ein bekanntes Gerät,

Fig. 2 ein erfindungsgemäßes Gerät,

Fig. 3 einen Querschnitt einer Seitenansicht eines Teils von Fig. 2,

Fig. 4 und 5 eine Draufsicht auf einen Querschnitt bei Fig. 3 und

Fig. 6 und 7 eine entsprechende Ansicht wie bei Fig. 4 und 5, jedoch mit einem Motorantrieb.

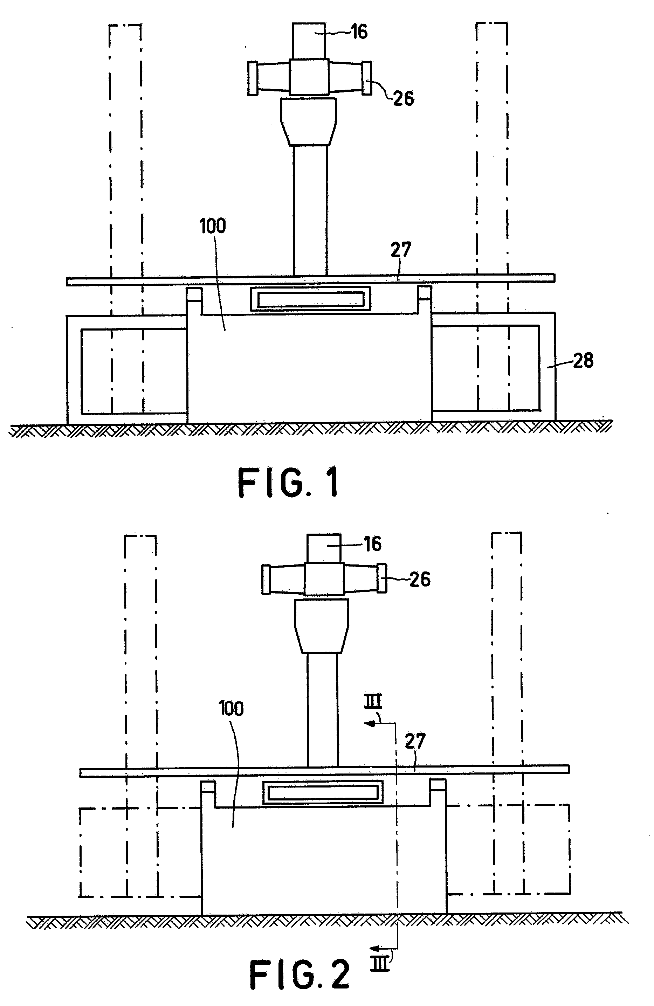

In Fig. 1 ist mit 27 der Patientenlagerungstisch bzw. dessen auf einem Grundgestell 100 angeordnete Tischplatte bezeichnet. Hinter dem Tisch ist eine Säule 16 angeordnet, die einen Röntgenstrahler 26 mit fest darunter angebrachter Tiefblende usw. trägt. Der Röntgenstrahler ist mittels einer rahmenförmigen Führungseinrichtung 28 in Tischlängsrichtung aus der ausgezogen dargestellten Mittelstellung in je eine seitliche Endstellung am Kopf oder Fußende verfahrbar, die strichpunktiert angedeutet ist. Der Rahmen, der eine Tiefe von ca. 20 cm hat, behindert den freien Zugang zur Tischplatte von der Längsseite aus, an der er angebracht ist, und zwar auch bei Untersuchungen, bei denen der Röntgenstrahler in der Mitte steht und die Verschiebbarkeit des Röntgenstrahlers gar nicht ausgenutzt wird.

Das in Fig. 2 dargestellte erfindungsgemäße Gerät unterscheidet sich von dem bekannten Gerät dadurch, daß in der Mittenstellung die Führungseinrichtung nicht seitlich an dem Grundgestell des Patientenlagerungstisches 27 vorbeiragt und nicht den Zugang zum Kopf- oder Fußende der Tischplatte behindert. Gleichwohl ist auch hier die Säule 16 mit dem Röntgenstrahler 26 zum Fuß- oder Kopfende hin verfahrbar, wie strichpunktiert angedeutet. In diesem Fall bewegt sich zwar auch die Führungseinrichtung mit zum Fuß- bzw. Kopfende, jedoch nicht auf dem Boden, so daß auch dann noch der Untersucher hinreichende Fußfreiheit beim Arbeiten am Gerät hat.

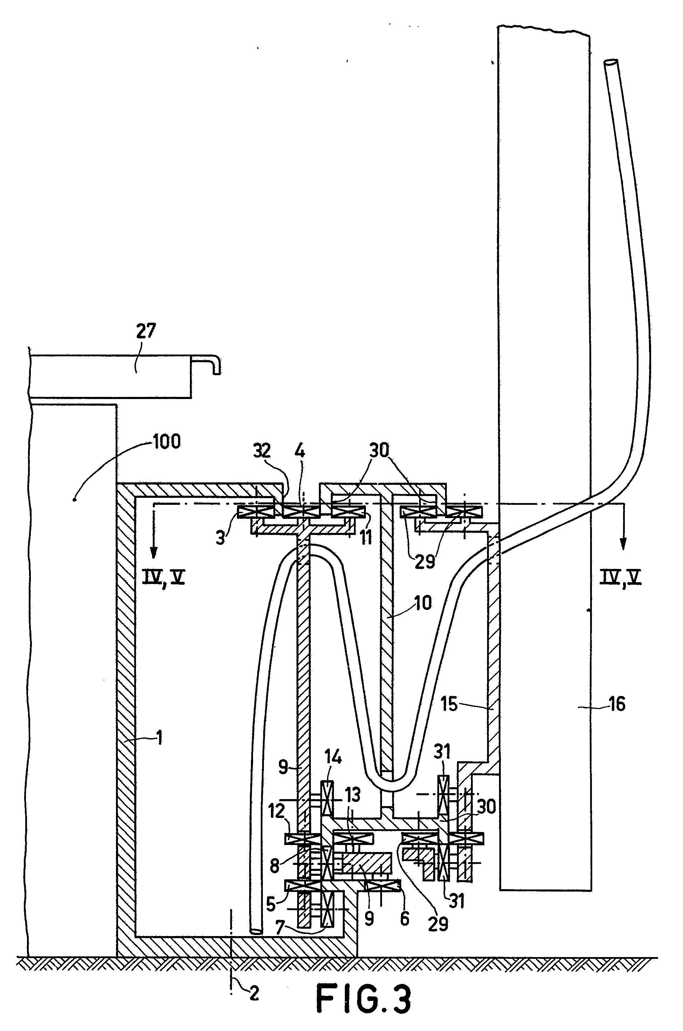

Fig. 3 zeigt die Führungseinrichtung in einer zur Tischplattenlängsrichtung senkrechten Ebene. Man erkennt, daß die Säule 16 an einem ersten Wagen 15 befestigt ist. Der erste Wagen 15 ist in Tischlängsrichtung entlang einer Führungsschiene 10 verschiebbar. Zu diesem Zweck ist der Wagen 15, der im wesentlichen durch ein Gestell aus Flachstahl gebildet wird, oben und unten mit Rollenpaaren 29 mit vertikaler Drehachse versehen, die auf den beiden sich senkrecht zur Zeichenebene erstreckenden Seitenflächen je eines Steges 30 an der Führungsschiene 10 laufen. Außerdem sind Rollen 31 mit horizontaler in der Zeichenebene liegender Drehachse am Wagen 15 befestigt, die auf horizontalen Stirnflächen an der Schiene 10 bzw. dem Steg 30 laufen, so daß das Gewicht des Wagens 15 bzw. der Säule 16 von der Führungsschiene 10 getragen wird. Da die Säule 16 ganz von dem Wagen 15 bzw. der Führungsschiene 10 getragen wird, braucht sie nicht auf dem Fußboden zu laufen. Sie kann daher 10 bis 20 cm über dem Fußboden enden.

Die Führungsschiene 10, die im wesentlichen über ihre gesamte Länge (in Richtung senkrecht zur Zeichenebene) den in der Zeichnung dargestellten doppel-T-förmigen Querschnitt mit senkrechten Stegen 30 aufweist, liegt auf den Laufflächen von Rollen 8 mit horizontaler Drehachse auf, von denen die Zeichnung nur eine zeigt (die andere liegt außerhalb der Zeichenebene dahinter), deren Drehachse in der Zeichenebene liegt. Die Rollen 8 laufen auf einem am Boden (bei 2) befestigten mit dem Patientenlagerungstisch verbundenen Rahmen 1. Dieser Trägt also die Führungsschiene 10 und mit ihr den Wagen 15, die Säule 16 und den daran befestigten Röntgenstrahler 26 (Fig. 2).

Die Rollen 8 sind an einem zweiten Wagen 9 befestigt, der ähnlich aufgebaut ist wie der erste Wagen 15. Er besitzt oben drei Rollenpaare 3, 4, 11 mit vertikalen Drehachsen, die voneinander einen solchen Abstand haben, daß dazwischen gerade noch die am Rahmen 1 bzw. an der Führungsschiene 10 befestigten Stege 32 bzw. 30 mit vertikalen Seitenflächen Platz haben. Dadurch wird ein bestimmter seitlicher Abstand zwischen dem Rahmen 1 und der Führungsschiene 10 erzwungen. Auch am unteren Teil des Wagens sind Rollenpaare 5 und 6 bzw. 12 und 13 mit vertikaler Drehachse befestigt, die auf entsprechenden Seitenflächen am Rahmen 1 bzw. an der Führungsschiene 10 laufen. Der seitliche Abstand aller Komponenten der Führungseinrichtung mit Einschluß der Säule 16 ist somit festgelegt. Weitere Rollen 14 und 7 sind vorgesehen, deren Drehachse parallel zur Drehachse der Rollen 8 verläuft und die oberhalb bzw. unterhalb der Rollen 8 am Wagen 9 befestigt sind. Zwischen diesen Rollen und den Rollen 8 läuft jeweils ein Teil der Führungsschiene 10 bzw. des Rahmens 1, so daß auch die Position der einzelnen Elemente der Führungseinrichtung in vertikaler Richtung festgelegt ist. Die Elemente können sich daher nur noch in Richtung senkrecht zur Zeichenebene gegeneinander verschieben.

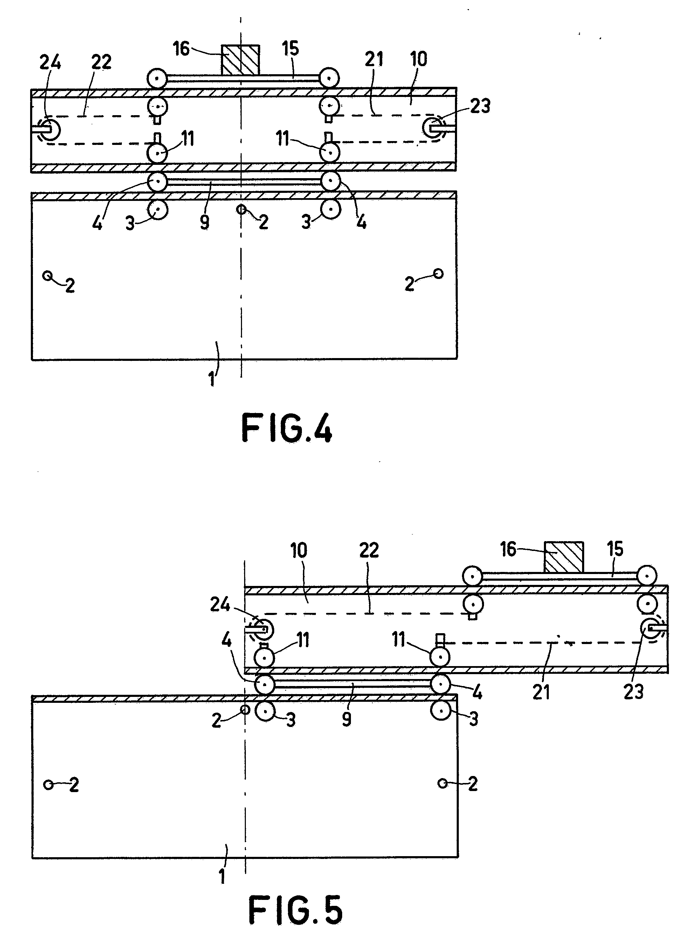

Wie die Fig. 4 und 5 zeigen, die eine Draufsicht auf einen Querschnitt längs der Linien IV, V in Fig. 3 — einmal in zentraler Stellung der Säule (Fig. 4) und einmal bei ganz zur Seite verfahrener Säule (Fig. 5) — darstellen, sind an den kopf- bzw. fußseitigen Enden der Führungsschiene 10 Umlenkrollen 23 und 24 befestigt. Um die Umlenkrollen 23 und 24 sind Seile (21, 22), Stahlbänder o. dgl. geführt und mit den zugewandten Enden der Wagen 15 und 9 verbunden. Dadurch wird erzwungen, daß sich die Wagen 9 und 15 nur gegensinnig in bezug auf die Führungsschiene 10 bewegen können, wobei jeder Wagen die gleiche Strecke zurücklegt.

Bei einer Verschiebung der Säule, z. B. nach rechts, passiert folgendes:
Der Wagen 9 verschiebt sich gegenüber dem Rahmen 1 um eine (zunächst als beliebig vorausgesetzte) Strecke a. Eine solche Verschiebung ist nur möglich, wenn die Rollen 4 und insbesondere die Rollen 8 auf den Laufflächen des Rahmens 1 abrollen, und ein solches Abrollen wiederum ist nur möglich, wenn sich die Führungsschiene um genau die doppelte Strecke nach rechts verschiebt wie der Wagen 9, d. h. um die Strecke 2a. Der Wagen 9 verschiebt sich also in bezug auf die Führungsschiene 10 um die Strecke a nach links. Aus den obengenannten Gründen verschiebt sich dann der über die Umlenkrollen 23 und 24 und die Riemen 21 bzw. 22 damit verbundene Wagen 15 gegenüber der Führungsschiene um die gleiche Strecke a, allerdings nach rechts. Die Gesamtverschiebung des Wagens 15 bzw. der Säule 16 ergibt sich dann aus einer Addition der Führungsschiene 10 — 2a und des Wagens 15 (in bezug auf die Führungsschiene 10) — a; d. h. die Gesamtverschiebung der Säule 16 bzw. des Wagens 15 nach rechts hin beträgt 3a — also das Dreifache der Verschiebung, die dabei der Wagen 9 erfährt.

Bei dieser Bewegung verschiebt sich also die Säule bzw. der Wagen 15 gegenüber der Führungsschiene 10, die Führungsschiene 10 gegenüber dem Wagen 9 und der Wagen 9 gegenüber dem Rahmen 1 jeweils um die gleiche Strecke a. Die zwangsläufige Kopplung zwischen der Bewegung der einzelnen Geräte wird dabei hauptsächlich durch die Rollen 8 gewährleistet, die das Gewicht der Führungsschiene 10, des Wagens 15 und der Säule mit dem Röntgenstrahler 26 tragen, weil die aufgrund dieser Belastung der Rollen auftretenden Reibungskräfte so groß sind, daß praktisch ausgeschlossen ist, daß der Wagen 9 gegenüber dem Rahmen 1 verschoben wird, ohne daß dabei die Rollen 8 auf dem Rahmen 1 ablaufen und die Führungsschiene 10 auf den Rollen in der gleichen Richtung aber um die doppelte Strecke mitrollt. Es ist daher praktisch ausgeschlossen, daß sich der Wagen 15 gegenüber der Führungsschiene 10 oder diese gegenüber dem Wagen 9 verstellt, während alle anderen Teile ihre Lage beibehalten. Beim Zurückschieben der Säule ergibt sich daher immer die gleiche Grundposition (Fig. 4) — Fig. 5 zeigt die Anordnung bei ganz nach rechts verschobener Säule.

Besonders zweckmäßig ist es, wenn die Führungsschiene 10 genauso lang ist wie der Rahmen 1, z. B. 1 m, und der Wagen 9 genauso lang wie der Wagen 15 und halb so lang wie die Führungsschiene 10 bzw. der Rahmen 1, d. h. 50 cm. Dann haben alle Teile gleichzeitig auf dem Teil, auf dem sie verfahren werden, ihre Endstellung erreicht. Die maximale Verfahrbarkeit in einer Richtung beträgt dann das Dreifache der halben Wagenlänge (75 cm). Bei einer Rahmenbreite von 1 m liegen also die Endstellungen der Säule 16 um 1,50 m auseinander.

Wie Fig. 4 zeigt, ist die Säule 16 in der Mitte des Wagens 15, der Wagen 15 in der Mitte der Führungsschiene 10, die Führungsschiene 10 mittig zum Wagen 9 und der Wagen 9 wieder in der Mitte des Rahmens 1 montiert. Durch diese symmetrische zentrierte Anordnung wird erreicht, daß die Verschiebung in beiden Richtungen symmetrisch erfolgt. Es ist aber auch eine asymmetrische Anordnung denkbar, so daß sich eine asymmetrische Verschiebung ergibt und die Säule in der einen Richtung weiter ausgefahren werden kann als in der entgegengesetzten Richtung.

Die gegensinnige Verschiebung der beiden Wagen gegenüber der Führungsschiene 10 kann auch auf andere Weise als in Fig. 4 und 5 dargestellt (mittels der Umlenkrollen 23, 24 und der Riemen bzw. Bänder o. dgl. 21, 22) erfolgen. Unter anderem könnte mit den beiden Wagen je eine in der Verschieberichtung verlaufende Zahnstange verbunden sein, die mit einem (oder mehreren) an der Führungsschiene 10 befestigten Zahnrädern zusammenwirken. Durch geeignete Über- oder Untersetzung könnte dabei auch erreicht werden, daß die gegensinnigen Bewegungen nicht im Verhältnis 1 : 1 erfolgen.

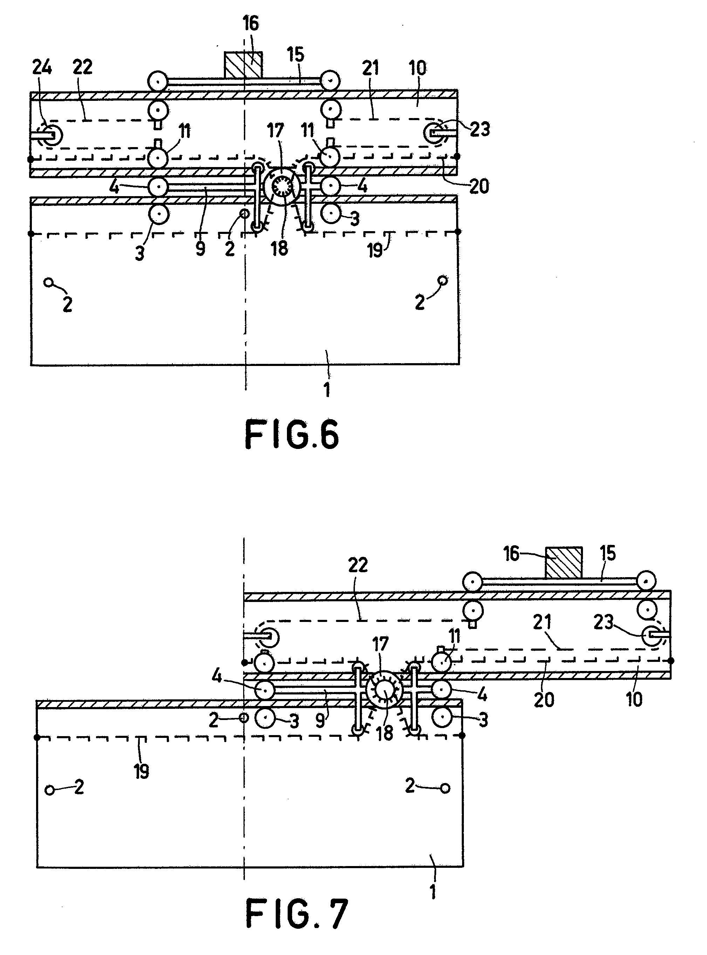

Die Kopplung des Wagens 9 mit der Führungsschiene 10, die sicherstellt, daß der Wagen und die Führungsschiene sich gegenüber dem Grundgerät im Verhältnis 1 : 2 bewegen, kann auch auf andere Weise erreicht werden als durch die Rollen 8 und 4. Beispielsweise könnten auch hier Umlenkrollen mit Seilen oder Zahnstangen und Zahnrädern oder Hebelübertragung benutzt werden. Die Fig. 6 und 7, die die gleichen Ansichten wie Fig. 4 und 5 — jedoch mit einem Motorantrieb — darstellen, zeigen eine weitere Möglichkeit, um diese Bewegungsverhalten zu erzwingen. Sie basieren auf der Verwendung eines Motorantriebes. An den (in Tischlängsrichtung) gegenüberliegenden Enden sowohl des Rahmens 1 als auch der Führungsschiene 10 ist je ein Zahnriemen 19 bzw. 20 befestigt. Jeder der beiden Zahnriemen 19, 20 ist gegenläufig um ein Zahnrad 18 geführt (die Zeichnung zeigt nur das vordere Zahnrad), die auf einer gemeinsamen zur Zeichenebene senkrechten Welle angeordnet sind, die von einem Motor 17 angetrieben wird. Wenn die Zahnräder 18 dabei im Gegenuhrzeigersinn angetrieben werden, zieht der Motor 17 sich und den Wagen 9, auf dem er befestigt ist, nach rechts und damit auch die Führungsschiene 10. Diese wird durch die Drehung des mit dem Zahnriemen 20 gekoppelten Zahnrades 18 zusätzlich nach rechts verschoben, so daß sie gegenüber dem Rahmen 1 den doppelten Weg wie der Wagen 9 zurücklegt. Die Wagen 9 und 15 sind genauso gekoppelt wie bei den Fig. 4 und 5, so daß auf eine erneute Beschreibung verzichtet werden kann.

Fig. 7 zeigt die Anordnung in der rechten Endstellung der Säule.

**Patentansprüche**

1. Röntgenuntersuchungsgerät mit einem Grundgestell (100), einer verschiebbar mit dem Grundgestell (100) verbundenen Patientenlagerungsplatte (27) und einer einen Röntgenstrahler (26) tragenden Säule (16), die längs einer sich in Tischlängsrichtung erstreckenden Führungsschiene (10) verfahrbar ist, dadurch gekennzeichnet, daß die Führungsschiene (10) in Tischlängsrichtung längs eines Wagens (9) verfahrbar ist, der ebenfalls in Tischlängsrichtung längs eines ortsfesten Rahmens (1) verfahrbar ist, daß zwischen der Führungsschiene (10) und dem Rahmen (1) wenigstens eine am Wagen (9) befestigte Rolle (4, 8) so angeordnet ist, daß bei einer Verschiebung der Führungsschiene (10) die Rolle (4, 8) zwangsläufig auf dem Rahmen (1) abrollt, und daß der Wagen (9) und die Säule (16) so miteinander gekoppelt sind, daß bei einer Verschiebung des Wagens (9) relativ zur Führungsschiene (10) die Säule (16) relativ zur Führungsschiene (10) gegensinnig um dieselbe Strecke verschoben wird.

2. Röntgenuntersuchungsgerät nach Anspruch 1, dadurch gekennzeichnet, daß die Rolle (8) um eine horizontale Achse drehbar ist und das Gewicht der Führungsschiene (10) trägt, die ihrerseits das Gewicht der auf ihr verfahrbaren Säule (16) trägt.

3. Röntgenuntersuchungsgerät nach einem der vorhergehenden Ansprüche, dadurch gekennzeichnet, daß an der Säule (16) bzw. an einem mit der Säule starr verbundenen Teil (15) Rollen (29, 31) befestigt sind, die auf der Führungsschiene (10) laufen.

4. Röntgenuntersuchungsgerät nach Anspruch 1, dadurch gekennzeichnet, daß am oberen Teil des Wagens wenigstens eine Rolle (4) mit vertikaler Achse vorgesehen ist, die bei einer Verschiebung der Führungsschiene auf der Oberfläche der Führungsschiene (10) und des Rahmens (1) abrollt, daß am oberen Teil des Wagens (9) wenigstens ein Paar weitere Rollen (3, 11) mit vertikaler Achse befestigt sind, und daß in den Zwischenräumen zwischen der Rolle (4) und je einer der Rollen (3, 11) des Paares je ein Steg (30, 32) des Rahmens bzw. der Führungsschiene (10) gleitend angeordnet ist, dessen Seitenflächen in zur Tischlängsrichtung parallelen Vertikalebenen verlaufen.

5. Röntgenuntersuchungsgerät nach einem der vorhergehenden Ansprüche, dadurch gekennzeichnet, daß an in Tischlängsrichtung gegeneinander versetzten Stellen der Führungsschiene (10) zwei Umlenkrollen (23, 24) vorgesehen sind und um je eine Umlenkrolle (23, 24) ein Seil, Riemen, Kette oder dergleichen (21, 22) geführt ist, das an je einem Punkt des Wagens (9) und der Säule (16) befestigt ist.

6. Röntgenuntersuchungsgerät nach einem der vorhergehenden Ansprüche, dadurch gekennzeichnet, daß auf dem Wagen (9) ein Motor (17) vorgesehen ist, der mit am Rahmen (1) und an der Führungsschiene (10) angeordneten Kraftübertragungselementen (19, 20) so gekoppelt ist, daß bei einer Drehung des Motors (17) der Wagen (9) in der einen Richtung und die Führungsschiene (10) in derselben Richtung, jedoch um die doppelte Strecke verschoben wird.

7. Röntgenuntersuchungsgerät nach einem der vorhergehenden Ansprüche, dadurch gekennzeichnet, daß die Führungsschiene (10) in Tischlängsrichtung etwa die gleichen Abmessungen hat wie der Rahmen (1), und daß der Wagen (9) annähernd halb so breit ist wie der Rahmen (1) bzw. die Führungsschiene (10).

**Claims**

1. An X-ray examination apparatus comprising a basic frame (100), a patient examination table (27) which is slidably connected to the basic frame (100), and a column (16) which supports an X-ray source (26) and which is movable on a guide rail (10) which extends in the longitudinal direction of the table, characterized in that the guide rail (10) is movable in the longitudinal direction of the table on a carriage (9) which is also movable in the longitudinal direction of the table on a stationary frame (1), there being arranged between the guide rail (10) and the frame (1) at least one roller (4, 8) which is connected to the carriage (9) so that when the guide rail (10) is moved, the roller (4, 8) is forced to roll on the frame (1), the carriage (9) and the column (16) being linked so that when the carriage (9) is moved relative to the guide rail (10), the column (16) is moved over the same distance but in the opposite direction relativ to the guide rail (10).

2. An X-ray examination apparatus as claimed in claim 1, characterizes in that the roller (8) is rotatable around a horizontal axis and supports the weight of the guide rail (10) which in turn supports the weight of the column (16) which is displaceable thereon.

3. An X-ray examination apparatus as claimed in any of the preceding claims, characterized in that rollers (29, 31) which run on the guide rail (10) are connected to the column (16) or to a part (15) which ist regidly connected to the column.

4. An X-ray examination apparatus as claimed in claim 1, characterized in that on the upper part of the carriage there is provided at least one roller (4) with a vertical axis, which roller rolls on the surface of the guide rail (10) and the frame (1) when the guide rail is displaced, and the upper part of the carriage (9) has connected to it at least one pair of further rollers (3, 11) with vertical axes, there being slidably arranged in each of the spaces between the one roller (4) and the further rollers (3, 11) a flange (30, 32) of the frame or the guide rail (10), the side faces of the flange each extending in a vertical plane parallel to the longitudinal direction of the table.

5. An X-ray examination apparatus as claimed in any of the preceding claims, characterized in that at locations on the guide rail (10) which are spaced from each other in the longitudinal direction of the table there are provided two guide pulleys (23, 24), there being arranged around each guide pulley (23, 24) a rope, belt, chain or the like (21, 22) which is connected to a point on the carriage (9) and the column (16).

6. An X-ray examination apparatus as claimed in any of the preceding claims, characterized in that on the carriage (9) there is provided a motor (17) which is coupled to force transmission elements (19, 20) which are arranged on the frame (1) and the guide rail (10) so that when the motor (17) rotates, the carriage (9) is desplaced in one direction and the guide rail (10) is displaced in the same derection, but over twice the distance.

7. An X-ray examination apparatus as claimed in any of the preceding claims, characterized in that the guide rail (10) has approximately the same dimensions in the longitudinal direction of the table as the frame (1), the width of the carriage (9) being approximately half the width of the frame (1) or the guide rail (10).

**Revendications**

1. Appareil d'examen à rayons X comportant un bâti de base (100), une table de support (27) coulissante pour les patients reliée au bâti de base (100) et une colonne (16) portant une source (26) de rayons X qui peut être déplacée le long d'un rail de guidage (10) s'étendant dans le sens longitudinal de la table, caractérisé en ce que le rail de guidage (10) est déplaçable dans le sens de la longueur de la table le long d'un chariot (9) qui est également déplaçable dans le sens de la longueur de la table le long d'une membrure fixe (1), entre le rail de guidage (10) et la membrure (1) est monte au moins un galet (4, 8) fixé au chariot (9), dans le cas d'un déplacement du rail de guidage (10), le galet (4, 8) roule forcément sur la membrure (1) et le chariot (9) ainsi que la colonne (16) sont couplés l'un à l'autre de telle façon que lors d'un déplacement du chariot (9) par rapport au rail de guidage (10), la colonne (16) soit déplacée de la même distance dans le sens opposé par rapport au rail de guidage (10).

2. Appareil d'examen à rayons X suivant la revendication 1, caractérisé en ce que le galet (8) peut tourner autour d'un axe horizontal et supporte le poids du rail de guidage (10) qui, pour sa part, supporte le poids de la colonne (16) qui se déplace sur sa surface.

3. Appareil d'examen à rayons X suivant l'une quelconque des revendications précédentes, caractérisé en ce que des galets (29, 31), qui roulent sur le rail de guidage (10) sont fixés à la colonne (16) ou à un élément (15) relié rigidement à la colonne.

4. Appareil d'examen à rayons X suivant la revendication 1, caractérisé en ce qu'à la partie supérieure du chariot est prévu au moins un galet (4) à axe vertical qui, lors d'un déplacement du rail de guidage roule sur la surface de ce rail de guidage (10) et de la membrure (1), à la partie supérieure du chariot (9) sont fixés au moins deux autres galets (3, 11) à axe vertical et dans les espaces intermédiaires entre le galet (4) et chacun des galets (3, 11) de la paire est disposée à glissement une aile (30, 32) de la membrure ou du rail de guidage (10), les faces latérales de cette aile se déplaçant dans des plans verticaux parallèles à la longueur de la table.

5. Appareil d'examen à rayons X suivant l'une quelconque des revendications précédentes, caractérisé en ce que deux galets de renvoi (23, 24) sont prévus en des endroits du rail de guidage (10) décalés l'un par rapport à l'autre dans le sens de la longueur de la table et un câble, une courroie, une chaîne ou un élément analogue (21, 22) passe autour de chaque galet de renvoi (23, 24) et est fixé chaque fois à un point du chariot (9) et de la colonne (16).

6. Appareil d'examen à rayons X suivant l'une quelconque des revendications précédentes, caractérisé en ce qu'un moteur (17) est prévu sur le chariot (9) et est accouplé par des éléments de transmission de force (19, 20) montés sur la membrure (1) et sur le rail de guidage (10) de telle façon que, dans le cas d'une rotation du moteur (17), le chariot (9) soit déplacé dans le premier sens et le rail de guidage (10) dans le même sens mais d'une distance double.

7. Appareil d'examen à rayons X suivant l'une quelconque des revendications précédentes, caractérisé en ce que le rail de guidage (10) a dans le sens de la longueur de la table à peu près les mêmes dimensions que la membrure (1) et le chariot (9) a une largeur égale environ à la moitié de celle de la membrure (1) ou du rail de guidage (10).

**FIG. 1**

**FIG. 2**

FIG.3

FIG.4

FIG.5

FIG.6

FIG.7